# EUROPEAN PATENT APPLICATION

(11) **EP 0 872 253 A2**
(43) Date of publication of application: **21.10.1998**
(21) Application number: 98302620.4
(22) Date of filing: 02.04.1998
(51) Int. Cl.: A61M 16/04, A61M 25/10

(54) **Cuffed medico-surgical tubes**

(30) Priority: 16.04.1997 GB 9707725
(71) Applicant: Smiths Industries Public Limited Company, London, NW11 8DS (GB)
(72) Inventor: Pagan, Eric, Hythe, Kent, CT21 6DA (GB)
(74) Representative: Flint, Jonathan McNeill

(57) **Abstract**

A tracheal or similar tube with an inflatable cuff 4 is reinforced in a region 41 towards the patient end of the cuff by spraying the same material as the cuff onto the outside of the cuff to form a pattern of spots 48 of material that stiffens the reinforced region.

## Description

This invention relates to medico-surgical tubes of the kind including a tubular shaft and an inflatable cuff.

Cuffed tracheal tubes comprise a tubular shaft having an inflatable cuff encircling the tube close to its patient end. The cuff is deflated during insertion and, once the tube has been correctly located in the trachea, the cuff is inflated to contact the inside of the trachea. This confines flow of gas in the trachea along the bore of the shaft and also helps retain the tube in the correct position. Because the lining of the trachea is very delicate, it is important that the cuff does not exert an excess or localized pressure on the trachea. For this reason, the cuff is usually made from a very thin, flexible film of plastics or elastomeric material. This can, however, create a problem because the flexible nature of the cuff, especially at low pressures, enables the shaft to move axially, laterally or angularly to a certain extent relative to the cuff. Axial movement can be caused by the changes in pressure across the cuff between inspiration and expiration, which can move the shaft repeatedly backwards and forwards leading to rubbing and possible tissue damage.

It is an object of the present invention to provide an improved medico-surgical tube and method of manufacture.

According to the present invention there is provided a medico-surgical tube of the above-specified kind, characterised in that the inflatable cuff has a region that is reinforced and a region that is unreinforced.

Preferably, the cuff is reinforced in a region adjacent where the cuff is attached with the shaft. The cuff may encircle the shaft towards the patient end of the shaft, the cuff being attached to the shaft at the patient end of the cuff and at the machine end of the cuff, the cuff being reinforced in the region of the patient end of the cuff. The cuff is preferably reinforced by a material applied to the surface, such as the outside surface, of the cuff. The material may be applied by spraying and is preferably the same as the material of the cuff. The reinforcement may be provided by a pattern of spots of material on the cuff.

The tube may be a tracheal tube, the cuff being arranged to seal with tissue in the trachea, or the tube may be a laryngeal mask, the cuff being arranged to seal in the region of the hypopharynx.

Several examples of cuffed tracheal tubes in accordance with the present invention, will now be described, by way of example, with reference to the accompanying drawings, in which:
- Figure 1: is a side elevation view of a cuffed endotracheal tube;
- Figure 2: is an enlarged sectional side elevation view of the patient end of the tube of Figure 1;
- Figure 3: is an end view of the tube along the line III of Figure 2;
- Figures 4 to 6: are end views of alternative tubes;
- Figure 7: is a side elevation view illustrating manufacture of a tube according to the invention;
- Figure 8: is an enlarged sectional side elevation view of the patient end of an alternative tube;
- Figure 9: is a perspective view of the patient end of the tube of Figure 8; and
- Figures 10 and 11: are sectional side elevation views of two alternative tubes.

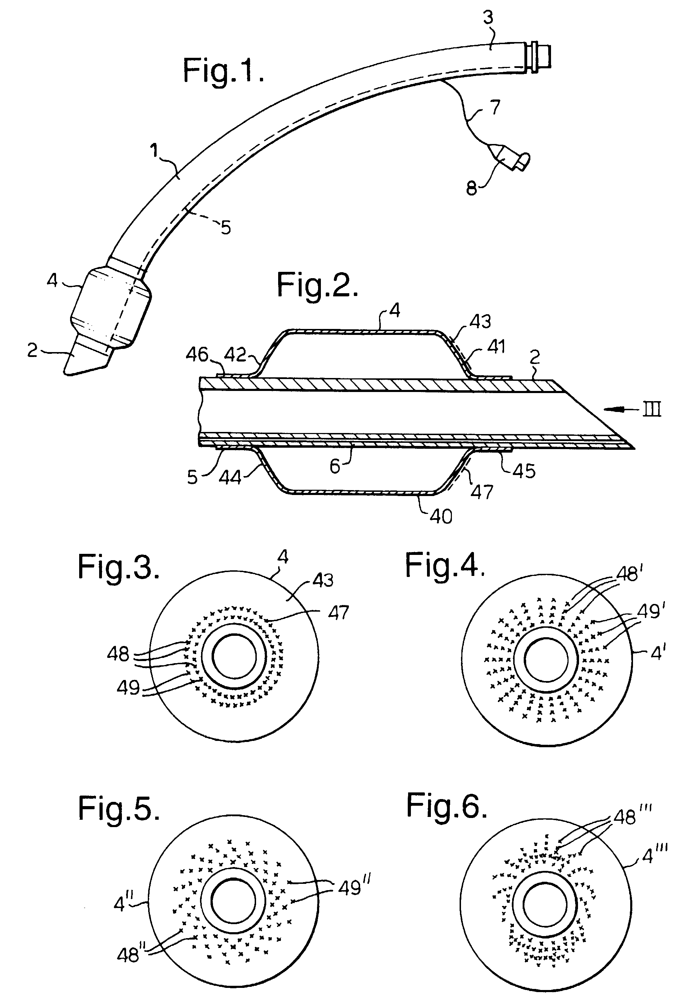

With reference first to Figures 1 to 3, the endotracheal tube has a curved tubular shaft 1 with a patient end 2 and a machine end 3. A cuff 4 encircles the shaft 1 close to the patient end and is inflated via an inflation lumen 5 so that the cuff seals with tissue in the trachea. The inflation lumen extends within the wall of the shaft and communicates with the inside of the cuff 4 through an opening 6 cut into the lumen through the outside wall of the shaft. Towards its rear end, the inflation lumen 5 is connected with an inflation line 7, which extends to a combined connector and inflation indicator 8.

The cuff 4 is blow moulded from a PVC or polyurethane material and is typically about 0.10mm thick. The cuff 4 is of tubular shape extending coaxially of the shaft 1. The cuff 4 has a central portion 40 of substantially cylindrical shape and about twice the diameter of the shaft 1, which, when inflated, contacts the surface of the patient's trachea. At opposite ends, the cuff 4 has a patient end portion 41 and a machine end portion 42. The end portions 41 and 42 both comprise a tapering region 43 and 44 adjacent respective sleeves 45 and 46, which are attached to the outer surface of the shaft 1 by a solvent or welding. The sleeves 45 and 46 extend in opposite directions outwardly of the central portion 40.

The tapering region 43 of the patient end portion 41 is reinforced by means of a coating 47 applied to its outer surface. The material of the coating 47 is the same as that forming the cuff 4 itself. The coating 47 is preferably not applied as a continuous layer but as a pattern of spots 48 of the coating material. The spots 48 may be arranged in various different patterns such as in concentric rings 49, as shown in Figure 3. Alternatively, the spots 48' could be arranged in straight lines 49' extending radially from the centre, as shown in Figure 4. In other arrangements, as shown in Figure 5, the lines 49" extend tangentially and may be straight or curved. In a further arrangement, as shown in Figure 6, the spots 48"' are arranged in combinations of patterns.

The reinforcing coating 47 increases the thickness and therefore increases the stiffness of the patient end portion 41 compared with the central portion 40 of the cuff. This has the effect of reducing the distance that a given force will displace the shaft 1 in the trachea relative to the central portion 40 of the cuff, which is fixed by contact with the surface of the trachea. Because only the end portion 41 is reinforced, the central region 40 can remain flexible, enabling an even pressure distribution over the surface of the trachea, thereby reducing the risk of trauma compared with a cuff that is stiffened along its entire length.

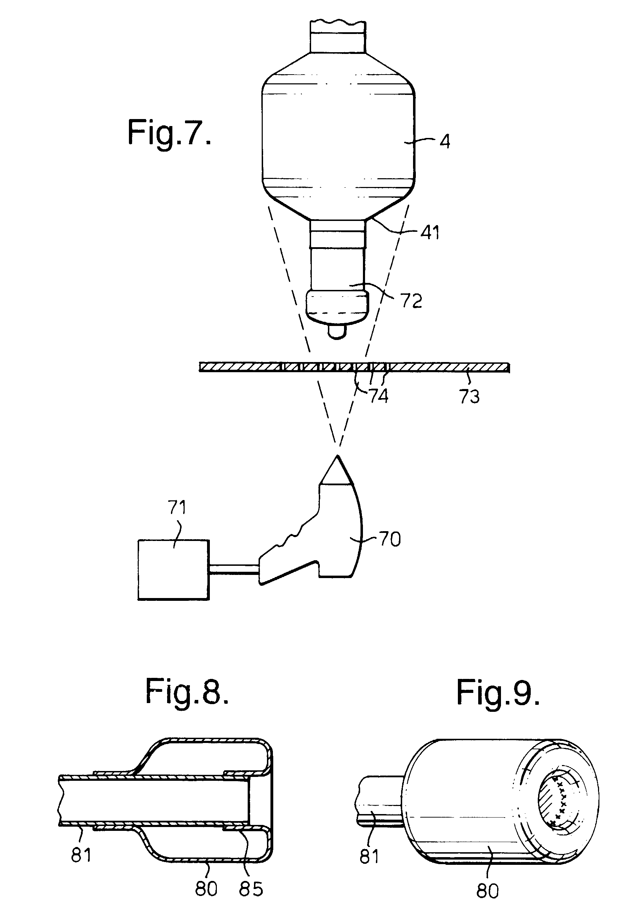

The tube is made by attaching the cuff 4 to the outside of the shaft 1, on top of the opening 6 from the inflation lumen 5, in the usual way. The cuff 4 is then inflated and the tube is placed patient-end on to a spray gun 70 connected to a source 71 of the reinforcement material, as shown in Figure 7. The patient end of the tube is closed with a cap 72. The pattern of the reinforcement spots is determined by a mask or template 73 located between the spray gun and the end of the tube. The template 73 has apertures 74 therethrough defining the location of the spots. Various different patterns could be produced by moving a template relative to the tube during spraying. After the spraying has been completed, the coated material is allowed to cure and the cuff is then deflated for sterilization and packaging, in the usual way.

Various alternative methods can be used to reinforce the cuff. For example, the reinforcement could be printed, laminated or dipped onto the cuff. The coating need not be a pattern of spots but could be a continuous film or layer. The reinforcement could be applied to the cuff before attaching it to the shaft. The reinforcement could be applied to the inside surface of the cuff by inverting the cuff after application; this has the advantage that the rougher surface produced by the reinforcement is located internally.

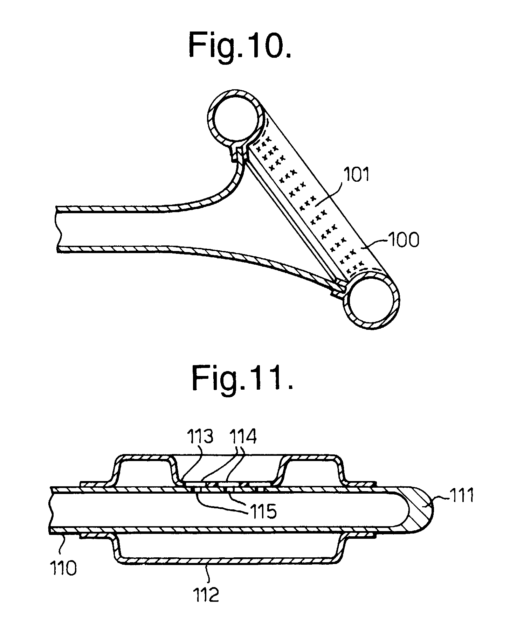

The reinforcement could be applied to cuffs of various different shapes such as the cuff 80 shown in Figures 8 and 9, which has its patient end sleeve 85 is everted internally within the cuff and the cuff projects a small distance beyond the end of the shaft 81. The reinforcement could be applied to cuffs on laryngeal masks (such as described in GB2317830) or oropharyngeal airways where the cuff seals with tissue in the region of the hypopharynx. An example of a laryngeal mask tube or airway is illustrated in Figure 10, the cuff 100 is of mask shape and is reinforced preferentially along the region 101.

Figure 11 shows a tube 110 with a closed patient end tip 111. A cuff 112, similar in shape to the cuff 40 shown in Figures 1 to 3, encircles the tube close to the closed tip. The cuff 112 has a region 113 in the middle of one side of the cuff, which is attached to the outside of the tube shaft and has apertures 114 formed therethrough aligned with apertures 115 through the wall of the shaft. The reinforcement could be applied to the patient end portion and, or alternatively, to the inflatable region adjacent the region 113 attached to the shaft.

## Claims

1. A medico-surgical tube including a tubular shaft (1) and an inflatable cuff (4, 80, 100, 112), characterised in that the inflatable cuff (4, 80, 100, 112) has a region (41, 101) that is reinforced and a region (40) that is unreinforced.

2. A tube according to Claim 1, characterised in that the cuff (4, 80, 100, 112) is reinforced in a region (41, 101) adjacent where the cuff is attached with the shaft (1).

3. A tube according to Claim 1 or in which the cuff (4, 80, 112) encircles the shaft (1) towards the patient end (2) of the shaft, the cuff (4, 80, 112) is attached to the shaft at the patient end (45) of the cuff and at the machine end (46) of the cuff, and characterised in that the cuff (4, 80, 112) is reinforced in the region (41) of the patient end (45) ofthe cuff.

4. A tube according to any one of the preceding claims, characterised in that the cuff (4, 80, 100, 112) is reinforced by a material applied to the surface of the cuff.

5. A tube according to Claim 4, characterised in that the material is applied to the outside surface of the cuff (4, 80, 100, 112).

6. A tube according to Claim 4 or 5, characterised in that the material is applied by spraying.

7. A tube according to any one of Claims 4 to 6, characterised in that the reinforcing material is the same as the material of the cuff (4, 80, 100, 112).

8. A tube according to any one of the preceding claims, characterised in that the reinforcement is provided by a pattern of spots (48) of material on the cuff (4, 80, 100, 112).

9. A tracheal tube according to any one of the preceding claims, characterised in that the cuff (4) is arranged to seal with tissue in the trachea.

10. A laryngeal mask according to any one of Claims 1 to 8, characterised in that the cuff (100) is arranged to seal in the region of the hypopharnyx.
